# EUROPEAN PATENT SPECIFICATION

(11) **EP 0 765 741 B1**
(45) Date of publication and mention of the grant of the patent: **07.06.2006**
(21) Application number: 96307060.2
(22) Date of filing: 27.09.1996
(51) Int. Cl.: B32B 27/08, B65D 75/36, A61F 2/16

(54) **Laminated barrier materials for the packaging of contact lenses**
Sperrschichtmaterialien für die Verpackung von Kontaktlinsen
Matériaux stratifiés barrière pour l'emballage de lentilles

(30) Priority: 29.09.1995 US 536160
(43) Date of publication of application: 02.04.1997
(73) Proprietor: Johnson & Johnson Vision Care, Inc., Jacksonville, FL 32216 (US)
(72) Inventor: Kindt-Larsen, Ture, 2840 Holte (DK); Martin, Wallace Anthony, Orange Park, Florida 32065 (US); Renkema, Kornelis, Jacksonville, Florida 32258 (US); Lust, Victor, Jacksonville, Florida 32257 (US)
(74) Representative: Mercer, Christopher Paul

(56) References cited:
- EP-A- 0 460 966
- EP-A- 0 613 772
- EP-A- 0 646 471
- EP-A- 0 650 676
- EP-A- 0 686 563
- EP-A- 0 691 270
- US-A- 4 691 820
- US-A- 5 084 356
- US-A- 5 337 888

## Description

The present invention relates to a cover-forming laminated plastic film packaging material for either an individual container or for interconnecting a plurality of containers, such as the base members of blister packages each of which is adapted to contain a hydrophilic contact lens in a sterile aqueous solution. More specifically, the invention pertains to a multi-layered clear or essentially transparent or translucent laminated barrier material constituting a covering label for the container or containers. The laminate is imprinted over at least portions of the surface extent thereof on either one or opposite sides of an outer or exposed layer covering the container or plurality of containers with a specific pattern of indicia and legends.

The packaging of hydrophilic contact lenses in a sterile aqueous solution is well known in the contact lens manufacturing technology. Particularly, packaging arrangements of that type generally consist of so-called blister packages which are adapted to be employed for the storage and dispensing of the hydrophilic contact lenses for use by a medical practitioner or a consumer who intends to wear the contact lenses. Such hydrophilic contact lenses, which may be disposable after a single wear or short-term use, are inexpensively manufactured from suitable hydrophilic polymeric materials; for example, copolymers of hydroxyethylene methacrylate (HEMA) containing from about 20% to 90% or more of water, depending upon the particular polymer composition. These contact lenses are generally stored in a sterile aqueous solution, ordinarily consisting of an isotonic saline solution, in order to prevent dehydration and to maintain the lenses in a ready-to-wear condition.

A container of the foregoing type normally comprises a base member which is molded from a suitable injection-molded or thermoformed plastic material; for instance, such as polypropylene, and incorporates a cavity adapted to house the contact lens in the aqueous solution, and which is sealingly closed by a label-forming cover, preferably in the form of a flexible multi-layered laminated material structure to provide a so-called blister package. This type of packaging arrangement has found widespread use in view of the inherently advantageous storing properties thereof and easy-to-dispense nature of the package by simply peeling the flexible laminated material from the base member, thereby enabling a user to gain ready access to the contact lens which is contained in the cavity of the base member. For example, a blister package which is adapted to provide a sterile sealed storage environment for a disposable, essentially single-use hydrophilic contact lens, which is normally worn for about 8 to 16 hours within a 24-hour period, wherein the lens is immersed in a sterile aqueous solution within the package is described in U.S. Patent No. 4,691,820 to Martinez.

In the above-mentioned U.S. patent, the blister package for storing and dispensing a hydrophilic contact lens includes an injection-molded or thermoformed plastic base portion or member incorporating a molded cavity which is surrounded by an outstanding planar flange extending about the rim of the cavity. A flexible cover sheet, such as a laminated foil is adhered to the surface of the flange so as to sealingly enclose the cavity in a generally liquid-tight manner. The surface of the covering foil may constitute a label and be imparted suitable printing indicia informative of the product stored in the blister package, the name and address of the manufacturer, and also incorporate various decorative designs and logos as desired; and also provide for changeable information, such as lot numbers, expiration dates and the like in addition to the foregoing, such as may be required by FDA regulations.

The foregoing blister packages are generally sold as individual or single units and the imprinted information provided thereon is complete for each blister package.

However, it is frequently intended to sell arrays or multiples of detachably interconnected of such blister packages, each containing respectively a single hydrophilic contact lens, suitably packaged in rigid containers or boxes housing predetermined quantities of such arrays of blister packages.

In that instance, a plurality of base members each having a cavity for containing a hydrophilic contact lens in a sterile aqueous solution are adapted to be positioned in a contiguous array and sealingly covered by a single or unitary flexible cover sheet, the latter of which is generally in the form of a multi-layered flexible foil laminate and is opaque in nature. The laminated foil may be provided with weakening lines intermediate each of the respective base members so as to enable individual segments of the foil member to be detached along the weakening lines and in conjunction with the therewith associated base member to be separated from the array when it is desired to gain access to the contact lens contained therein. This type of arrayed multiple interconnected blister package structure enables the compact packaging of a plurality of such arrays, each possessing a specified number of contact lens-containing base members interconnected by a single flexible cover sheet, within the confines of a suitable container, such as a rigid paperboard carton. In the carton there may be compactly stored a plurality of nested superimposed arrays of blister packages; for example, each array having five interconnected blister packages each having a single disposable contact lens housed therein. The carton may store six superimposed arrays of blister packages, for a total of thirty; or in effect, a 30-day supply of contact lenses for respectively one eye of a user.

The blister packages which are formed by means of this structure comprise a plurality of contiguously arranged thermoformed or injection-molded base members each containing a cavity for housing a hydrophilic contact lens in a sterile aqueous solution, and wherein the resultant array of such base members; for example, five (5) base members, is adapted to be sealingly covered and interconnected by a single multi-layered flexible laminated foil which also forms a common label.

The multi-layered laminated foil includes an outer layer of a plastic film material of a suitable polyolefin or preferably but not necessarily polyester, which is adhesively bonded to the surface of a supporting metallic foil, which may consist of aluminum, and in which the outer layer is double-sided printed; in effect, on both opposite surfaces thereof. The surface of the outer plastic film layer which faces towards and is adhered to the metallic foil is imprinted with suitable indicia and legends which may consist of permanent information regarding the manufacturer and the product, logos, instructive material, and decorative and advertising indicia relative the product in the blister package; whereas the opposite or exterior surface of the outer plastic film material layer may include suitable changeable information, such as expiration dates, lot numbers, fitting parameter, and other data specific to the packaged product. The interior surface of the outer plastic film material layer may be imprinted through the intermediary of suitable lithographic printing, either in single color or multi-colors and also provided with an appropriate printed background; whereas the changeable information specific to the product which is imprinted on specific areas of the outwardly facing surface of the outer film layer, may be printed thereon through thermal transfer printing. Adhered to the opposite surface of the metallic foil is a plastic film layer constituted of a polypropylene, which is adapted to be adhesively sealed to the base member of the blister package, which is also constituted of a compatible polypropylene composition.

Although the foregoing multi-layered laminated foil material has proven generally satisfactory in use, particularly in its application for the covering and sealing of the base member of the blister package containing the contact lens, upon occasion various problems can be encountered in connection with the use of a metallic foil as a constituent of the laminate. Thus, for example, the metallic foil, which is generally constituted of aluminum or the like, may cause excessive sealing or so-called "over sealing" and also a large variation in the peeling force required in attempting to peel the cover from the base member in order to gain access to the contact lens contained in the latter. Moreover, in the adhesive attachment of a laminated foil material, there has been produced a cover structure for the base member which may be subject to wrinkling, curling and bowing so as to essentially bend the blister package out of its intended configuration, particularly during heat sealing when the metallic foil acts as a so-called heat sink.

In order to improve upon the foregoing structure in providing a laminate which is a label and flexible cover for the base member of a blister package employed for the containment of a contact lens, rather than employing a laminated foil, pursuant to the invention the cover is of a clear or essentially transparent or translucent nature and is constituted primarily of adherent layers of clear plastic film which produces a more stable and lighter package at lower costs, and resultingly a cover structure less subject to wrinkling, curling and bowing of the blister package, while possessing a more uniformly controlled peeling force.

The present invention is directed to a clear laminated plastic material structure as defined in claim 1. Said material structure has a first clear plastic layer imprintable with a specified pattern of indicia and legends, a second clear plastic layer which is adhesively fastenable to a base member of the container and a barrier layer on at least one of said first and second clear plastic layers and adhesively fastened thereto to form said laminated material structure.

The present invention is further directed to a packaging arrangement as defined in claim 12 for the sealed containment of a plurality of hydrophilic contact lenses each in a sterile aqueous solution; comprising:
a) a plurality of molded plastic base members contiguously positioned in an array, each said base member having a cavity for containing one contact lens immersed in said aqueous solution, and each said base member including a substantially planar flange extending outwardly about the periphery of said cavity; and
b) a unitary generally transparent flexible cover sheet extending superimposed over said plurality of base members and being detachably sealed to the surface of said flanges at least about the rim of each said cavity, said cover sheet comprising a transparent outer layer imprinted to define a specified pattern of indicia and legends extending spread over the surface thereof, said laminate being separable into segments each forming a cover for respectively one therewith associated base member, whereby separation of at least one said segment form the remaining laminate provides visual evidence of separation from said array upon inspection of said printing pattern.

Pursuant to the invention, the laminated clear plastic film structure is essentially, although not necessarily, constituted of a basic three-ply construction possessing an inner layer capable of compatible heat sealing with the base member of the blister package; in essence, a polypropylene film which can be readily heat sealed to the generally polypropylene base member containing the contact lens; an outer layer constituted of an imprintable clear plastic film, preferably, although not necessarily, of a polyester composition; and an intermediate or center layer constituted of silicon oxide (SiO₂) which imparts suitable barrier and strength properties inhibiting the transmission therethrough of oxygen, water, bacteria and the like so as to maintain the quality and integrity of the contact lens contained in the blister package, while also maintaining a sterile environment internally thereof.

Alternatively, the barrier layer may be applied to the exterior surface of preferably the outer plastic film layer, rather than being interposed between the plastic film layers.

An additional advantage which is attained by the use of a clear plastic film laminate, in addition to the lighter weight package and resultant lower cost of manufacture, and the inherently improved stability of the plastic film laminate, resides in that the clear or essentially transparent or translucent nature of the film enables inspection of the contact lens contained in the base member subsequent to the sealing of the package. Moreover, the essentially clear cover construction, due to the use of the plastic film laminate in the absence of any metallic foil, also facilitates the additional processing through the sealed container; for example, the transmission of energy such as ultraviolet (UV) light into the container for the purpose of sterilizing the contents therein.

A further advantage which is derived by the elimination of any metallic foil resides in that the inherently plastic material nature of the packaging arrangement is more compatible and protective of the environment through the utilization of a silicon oxide barrier material.

As indicated hereinabove, while the silicon oxide layer interposed between or applied to the polypropylene film and the polyester film layer provides the barrier and strength properties, the polyester layer is printable through methods well known in the technology as referred to hereinabove and detailed in the further aspect hereinbelow. Moreover, the polypropylene film, which is compatible with the thermoformed or injection-molded base member, which is also constituted of polypropylene, can be readily heat sealed thereto and does not necessitate the need for any adhesives.

Pursuant to another aspect of the invention, suitable tie coats of a clear nature, such as clear polyurethane, may be adhesively interposed between the silicon oxide barrier material and the respective layers of polyester and polyethylene so as to form the multi-layered plastic laminate.

Accordingly, the present invention is directed to a novel clear plastic film laminate which is adapted to be utilized as a cover and label material for containers adapted to receive hydrophilic contact lenses in a sterile aqueous solution.

The present invention is further directed to a multi -layered clear plastic laminate adapted to provide a cover structure for blister packages containing contact lenses wherein an outer exposed layer of the laminate is adapted to be imprinted in a suitable manner; while a further film layer thereof is adapted to be heat sealed to at least one base member containing a contact lens in a sterile aqueous solution.

Still further the present invention is directed to a multi-layered clear plastic film material adapted to form a cover structure for one or more containers housing hydrophilic contact lenses, and which incorporates a silicon oxide barrier layer to provide a barrier against the transmission of oxygen, water, bacteria and the like for protecting the quality of the contact lens in maintaining a sterile environment within the sealed blister package.

Even further, the present invention is directed to a clear multi-layered plastic film cover structure incorporating a silicon oxide barrier layer for the packaging of contact lenses enabling the treatment through the sealed container of the sealed product by means of the transmission of energy, such as ultraviolet light, for the purpose of sterilizing the contents contained therein.

The features of the present invention may be more readily ascertained from the following detailed description of a preferred embodiment thereof, taken in conjunction with the accompanying drawings; in which:
Figure 1 illustrates a sectional view through a portion of a multi-layered clear plastic film construction pursuant to the invention;
Figure 2 illustrates a perspective view portion of the laminated clear plastic film material utilized as a covering for an array of blister packages, illustrating the imprinting on the outer layer of laminate;
Figure 3 illustrates a perspective view of the multi-layered clear plastic film laminate being disposed as a label and cover structure for an array of contiguously arranged and detachably interconnected base members for the formation of a plurality of contact lens-containing blister packages.
Figure 4 illustrates a perspective view of one of the blister packages shown as having been separated from the array of Fig. 3; and
Figure 5 illustrates the base member of the blister package of Fig. 4 with the sealing cover sheet of the package having been stripped off so as to facilitate access to a contact lens contained in a cavity formed in the base member of the blister package.

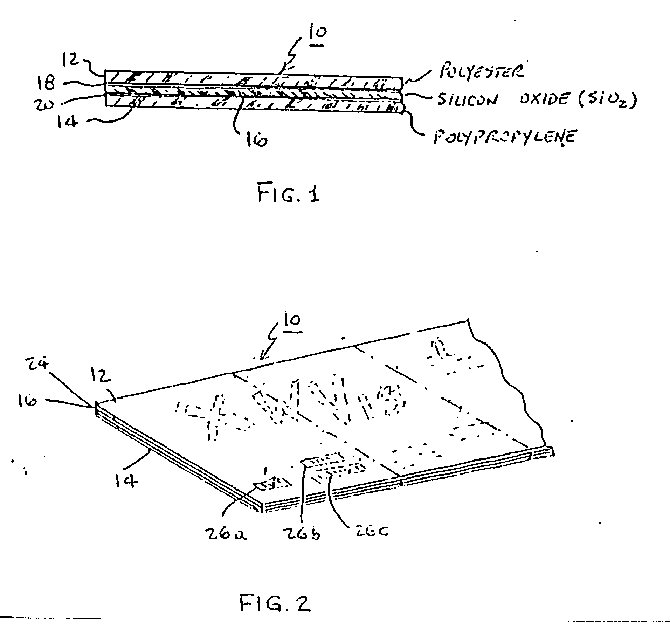

Referring now in more specific detail to the drawings, and particularly to Figures 1 and 2, there is illustrated a multi-layered laminate structure 10 essentially comprising a flexible cover sheet for base members adapted to sealingly house contact lenses in a sterile aqueous solution.

In this exemplary embodiment, as illustrated in Figures 1 and 2, the flexible cover sheet 10 comprises a plurality of superimposed layers of plastic film material possessing either a transparent or translucent nature so as to provide a substantially clear and light-transmissive cover and label structure for a blister package which is to be produced, consisting of the combination of the flexible cover sheet 10 and a base member containing the contact lens as described hereinbelow. In this instance, the flexible cover sheet 10 includes an outer or first layer 12 preferably constituted of a transparent or translucent imprintable polyester film and an inner or second layer 14 of a further plastic film, preferably constituted of a polypropylene adapted to be heat-sealed to contacting surface portions of the base member or base members, which is or which are constituted of a compatible thermoformed or injection-molded polypropylene. Interposed between the first plastic film layer 12 constituted of polyester and the second plastic film layer constituted of polypropylene is a barrier layer 16 constituted of silicon oxide (SiO₂), and the latter of which may be adhesively fastened to the contiguous or contacting surfaces of the respective polyester and polypropylene films 12, 14 through the interposition of suitable tie coats 18, 20; for example, which as clear polyurethane or the like.

It is also within the scope of the invention to apply the barrier layer 16 to the exterior surface of the first plastic film layer 12 rather than interposing the barrier layer 16 between the first and second plastic films 12 and 14.

The utilization of the barrier layer 16 constituted of silicon oxide rather than the heretofore or previously employed metallic foil material, such as aluminum foil, renders the entire cover sheet structure 10 essentially transparent or translucent, lighter in weight and of lower cost in its manufacture. Moreover, the elimination of the metallic foil renders the laminated plastic film structure 10 more stable, and consequently provides a flexible cover sheet construction less subject to being wrinkled, curled and bowed upon forming the blister package, since the heat adhesion of the metallic foil at times caused over-sealing or excessive sealing with a large variation in the peeling force which is required to remove the flexible cover sheet from the base member, in view of the aluminum acting as a heat sink during the heat sealing of the flexible cover sheet to the base member during the formation of the sealed blister package; while concurrently providing a more environmentally-protective packaging arrangement.

Moreover, the clear plastic laminate cover construction 10 also enables for additional processing or treatment of the contents subsequent to sealing the laminated plastic cover sheet to the base member in order to produce the sealed blister package, in that it enables transmission therethrough of energy such as ultraviolet light into the base member for the purpose of sterilizing the contents, in this instance, the contact lens contained in the cavity thereof; while concurrently facilitating visual inspection of the package contents through the clear cover 10.

The intermediate or center barrier layer 16 constituted of silicon oxide forms a barrier to the transmission therethrough of oxygen, water and bacteria or the like, thereby protecting the contact lenses in their sealed environment within the blister packages.

The utilization of the first layer 12 constituted of a polyester enables the latter to be readily imprinted on both sides thereof prior to being laminated to the barrier layer 16 so as to provide the required information thereon commensurate with the product contained in the blister package or packages.

The substantially transparent or translucent polyester film material constituting the first layer 12 of the plastic laminate 10 incorporating the silicon oxide barrier 16 may have been previously imprinted in a suitable manner as is conventional in the technology. For example, as illustrated in Figure 2 of the drawings, the flexible cover sheet 10, which as is illustrated, may be a continuous cover sheet for a plurality of adjacently located base members each containing respectively one contact lens immersed in a sterile aqueous solution, and through suitable lithography may be imparted on its lower surface 24 with suitable indicia which may be in solid or colored halftones, including reversely printed legends as required. Inasmuch as the indicia of halftones on the first layer 12 are printed on the interior surface 24, adjacent the silicon oxide barrier layer 16, upon viewing from the exterior facing outside towards the outer surface of the first layer 12, as shown in Fig. 2 of the drawings, in view of the transparency of the polyester film, visibly spread over the surface of the plastic laminate 10 are the various indicia and legends which may be representative of the company name, logo, various decorative and advertising matters and printed information which is to be permanently provided for products of this type in general, as desired. Additionally, suitable print fields 26a, 26b and 26c may be clearly ascertained and viewed from the exterior of the first film layer 12. Imprinted onto the outer or exposed surface of the first layer 12 in superposition over the various print fields 26a, 26b and 26c, may be the necessary information as required by FDA regulations specific to the lot, batch number, fitting parameter and lens power applicable to the product or contact lens contained in the blister package. This printing, as mentioned hereinbefore, is imparted to the interior or lower surface 24 of the first layer 12 in that indicia and legends are applied through lithographic printing in suitable orientation such that upon adhesive securing or bonding thereof to the silicon oxide barrier layer 16, the imprinted indicia will be viewable through the first layer 12 from the top or outside of the laminate 10 in the correctly represented pattern thereof.

The indicia may be in solid or colored halftones, including reversely printed legends as required, and which may also include a further color or print layer there beneath forming a colorated or contrasting background, for instance, such as white or any other suitable color. Inasmuch as the in indicia of halftones and the background beneath the first layer 12 are printed on interior surface 24, upon viewing from the outside towards the outer surface 10 of the first layer 12, as shown in Fig. 1 of the drawings, in view of the transparency of the polyester film, the various indicia and legends which may be representative of the company name, logo, various decorative and advertising matters and printed information which is to be permanently provided for products of this type in general. While conventional lithography is used in the preferred embodiment, it should be noted that other forms of printing processes could be utilized.

Prior to the forming of the adhesive bond between the first layer 12 and the adhesive 18 in order to laminate the first layer 12 to the barrier or silicon dioxide layer 16, suitable imprinting is implemented. This printing as mentioned hereinbefore, is imparted to the lower surface of the first layer 12 in that indicia and legends, as mentioned hereinbefore, are applied through halftone color printing in suitable orientation such that upon adhesive securing or bonding thereof to the barrier layer 16, the indicia will be viewable through the first layer 12 from the top of the laminate 10 in the correctly represented orientation thereof, as may be the case in the above-mentioned U.S. Patent No. 4,691,820.

As the laminated foil 10 is advanced guided over a series of rollers towards a printing head arrangement, an optical sensor unit scans and determines the location of at least one of the print fields 26a or 26b or 26c printed on the lower surface of the first layer 12, so as to cause the printing head arrangement to advance a ceramic printing head into contact with the applicable printing fields 26a through 26c, and through the intermediary of thermal transfer printing, imprint appropriately variable and changeable printed data to the exterior surface of the first layer 12. Such data, for example, when the laminate is severed into lid-forming labels for respective packages, may consist of suitable changeable lot numbers, expiration dates, and other physical data representative of the specific product housed in the package; for instance, data with regard to the power of a contact lens which is stored within a cavity of the package while immersed in a suitable protective sterilized saline solution.

Thus, the initial printing of the permanent indicia and legends which are provided on the lower surface of the first layer 12, which surface is adhesively bonded to the barrier or silicon oxide layer 16, and serves as a permanent display of the various generalized product-identifying and company-related information, advertising and logos or the like, while concurrently having at least one of the print fields serving as a register for imprinting by means of thermal transfer printing, other specific and variable product-related data onto the external surface of the foil laminate 10.

Although the foregoing has been described in connection with a flexible cover sheet 10 which is constituted of the clear or essentially transparent or translucent multi-layered plastic layers 12, 14 incorporating therebetween a silicon oxide barrier layer 16, and wherein the description discusses initially the use thereof for a single or individual blister package comprising a single base member which, respectively houses a single contact lens immersed in a sterile aqueous solution, it is readily contemplatable that a sheet of the flexible plastic cover material 10 may concurrently be commonly superimposed over a plurality of contiguously or adjoiningly arranged base members for the formation of an array or plurality of blister packages.

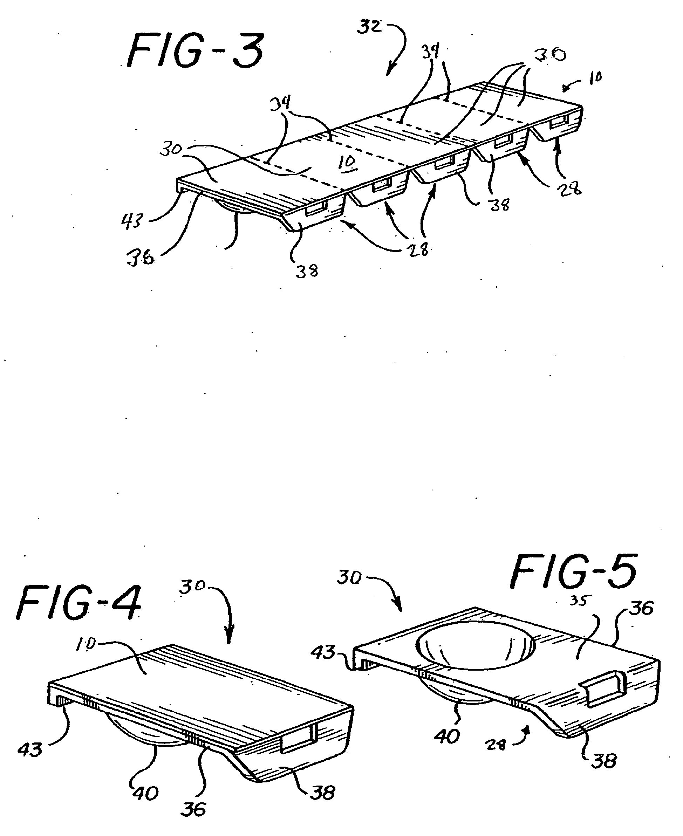

Hereby, as shown in Figure 2 the lines of separation between adjacent base members for the formation of respective blister packages may be continuous slits to separate the flexible cover sheet 10 into individual segments forming a cover and label for an individual blister package; or alternatively, as shown more clearly in Figure 3, may be only provided with discontinuous weakening lines in order to form an interconnected array of blister packages adapted to be subsequently separated as required by a user.

In the event that the plastic laminate cover sheet 10 is adapted to be positioned over a plurality of base members so as to form an array of a predetermined number of interconnected blister packages, this may be ascertained from Figure 3 of the drawings.

Positioned to extend over the base members 28 of a plurality or array 32 of blister packages 30, in this particular instance forming an array 32 of five (5) packages (30), is the label-forming and imprinted flexible clear plastic cover sheet 10, as shown in detail in Fig. 3, having a series of parallel spaced weakening lines 34, such as perforations, discontinuous slits or the like, provided to extend between each of the adjacently located base members 28. This enables suitable detachment from the array 32 of individual or single blister packages 30 each containing a single contact lens without affecting the integrity of the remaining blister packages, by tearing the laminate 10 along a weakening line 34, in accordance with the need of a user. The flexible clear laminate cover sheet 10 is adhesively fastened to suitable regions of the surface of flanges 35 on the base members 28 of the blister packages 30 facing the second layer 14 of the cover sheet 10, such as by heat sealing, so as to at least sealingly encompass each cavity containing a contact lens immersed in the sterile aqueous solution. Other sealing locations may also be provided at locations as desired between the surface of the second layer 14 of the flexible cover sheet 10 facing the surface of the flanges 35 so as to provide adequate regions of adherence therewith, while permitting various portions between these components 10, 38 to remain unattached to thereby facilitate finger-gripping engagement for separating or peeling off the severed segment of cover sheet 10 from the detached base member 28 in order to gain access to the contact lens which is contained in the respective cavity thereof.

In essence, Fig. 3 illustrates the array of packaging arrangements 30 as consisting of an array 32 of a plurality of interconnected blister packages 30, wherein each blister package 30, as represented in further detail in Figs. 4 and 5 of the drawings, includes a base member 28 consisting of a planar essentially rectangularly-shaped flange 35 having an integral depending wall portion 38 at one edge thereof. Offset towards an opposite edge of the flange 35 is a cavity 40 is formed therein which is of an essentially semispherical configuration, generally in conformance with the curvilinear shape of a contact lens (not shown), adapted to be stored therein in a sealed condition while immersed in a suitable sterile aqueous solution. However, other cavity configurations also readily lend themselves to the invention, such as semi-spherical, oval, or the like. Similarly the edge of the flange 35 opposite that possessing the depending wall portion is also provided with depending protuberances 43 which will also aid in positioning the blister packages in a carton.

The base member 28 of each blister package is constituted from an injection-molded or thermoformed plastic sheet material; for instance, such as polypropylene, in a manner similar to that described in U.S. Patent No. 4,691,820 to Martinez; which is assigned to the common assignee of the present application.

The foregoing clear or essentially transparent film and silicon oxide barrier laminate, in addition to providing the advantages as described hereinabove, may also have the first layer 12 suitably imprinted when utilized to provide an interconnected array of detachable blister packages so as to provide added protection to the integrity of the product contained therein.

Pursuant to the foregoing, in order to thwart potential diverters, the pattern of the permanently printed information on the lower or interior surface 24 of the first layer 12 which faces towards the silicon oxide barrier layer 14 may be spread across the length of the laminated cover sheet 10 extending substantially the full length of the array 32 whereby specific information, such as manufacturer's addresses and names, logos, product information of a permanent nature and the like will overlap or extend across the weakening lines 34 formed in the flexible laminated cover sheet 10; in effect, extend on both sides thereof above adjacent base members forming contiguously arranged blister packages in conjunction with the overlying label-forming laminated cover sheet, whereby separation of any particular blister package 30 by detaching along a weakening line 34 will provide visual information to a purchaser that the product has been potentially unlawfully converted so as to represent a similar, but essentially more expensive product, such as a reusable rather than disposable contact lens. The pattern of printing information of a permanent nature which is not easily accessible from the exterior of the laminated cover sheet 10, inasmuch as this printing is on the interior surface 24 of the outer or first layer 12 which is adhesively fastened or bonded to the silicon oxide barrier layer 16, can only be tampered with by destroying the cover sheet 10. Thus, when separated, the individual package will no longer be in conformance with applicable regulations inasmuch as the complete identity of the manufacturer will no longer be on the separated blister package. This, in effect, will then quite readily provide a tamper-proof contact lens blister package 30, in which the specially imprinted and configured label in the shape of the laminated cover sheet 10 is intended to thwart potential tamperers.

The changeable information with regard to the product contained in each blister package, such as lot numbers, expiration dates and contact lens power, may be separately printed within the printing areas 26a, 26b and 26c on the external or exposed surface of the first layer 12.

The foregoing structure is easily and completely packagable in cartons in a high-volume mass-production process. Moreaver, the invention facilitates the user-friendly handling and storage of large numbers of contact lenses by physicians and patients in a highly organized and convenient manner.

While there has been shown and described what are considered to be preferred embodiments of the invention, it will, of course, be understood that various modifications and changes in form or detail could readily be made without departing from the scope of the invention as hereinafter claimed.

## Claims

1. A clear laminated plastic material structure (10) comprising labels and sealing covers for a plurality of plastic containers (28) contiguously positioned in an array for sealing a product within each of said containers (28), said material structure (10) having a first clear film plastic layer (12) constituted of polyester or a polyolefin which is imprinted with a specified pattern of indicia and legends; a second clear film plastic layer (14) of polypropylene or polyethylene which is adhesively fastenable to a base member of each of the containers; and a barrier layer (16) of silicon oxide on at least one of said first and second clear plastic layers (12, 14) and adhesively fastened thereto to form said laminated material structure (10).

2. A laminated structure (10) as claimed in Claim 1, wherein said barrier layer (16) is interposed between said first and second clear plastic layers (12,14).

3. A laminated structure (10) as claimed in Claim 1 or claim 2, wherein said first layer (12) is an outer polyester film material layer and it has a surface (24) adhesively fastenable to a facing surface of said barrier layer (16), said pattern of specified indicia and legends being imprinted on the surface (24) of said polyester film material (12) facing said barrier layer (16).

4. A laminated structure (10) as claimed in Claim 1, Claim 2 or Claim 3, wherein said first film layer (12) is imprinted on both surfaces thereof, an outer surface of said first film layer (12) opposite said first imprinted surface being imprinted with further indicia at specific regions thereof.

5. A laminated structure (10) as claimed in Claim 4, wherein said further indicia is imparted to the outer surface of said first plastic film layer (12) by thermal transfer printing.

6. A laminated structure (10) as claimed in any one of the preceding claims, wherein said clear laminated plastic material structure (10) commonly forms said label and sealing cover for said array consisting of a plurality of contiguously arranged said containers (28); weakening lines (34) being formed in said laminated material structure (10) to facilitate separation into segments each consisting of a single container (28), said pattern of indicia and legends being printed on the material structure (10) extending over said weakening lines (34) such that predetermined regions of said printing are divided at said weakening lines (34) to form indication of discontinuities in said printing pattern upon separation of said segments.

7. A laminated structure (10) as claimed in Claim 6, wherein said weakening lines (34) each comprise perforations extending at least partially through said laminated material structure (10) to enable separating said structure into said segments along said weakening lines (34); said segments each forming a cover for respectively one said container (28).

8. A laminated structure (10) as claimed in Claim 6 or Claim 7, wherein said array of contiguously arranged containers (28) comprises a linear array consisting of a specified number of said containers (28), said laminated material structure (10) having a generally rectangular configuration for covering said array.

9. A laminated structure (10) as claimed in Claim 6, Claim 7 or Claim 8, wherein said printing pattern of indicia and legends is configured to form a protective printing pattern inhibiting misrepresentation of product in said containers (28).

10. A laminated structure (10) as claimed in any one of the preceding Claims, wherein said printing pattern is applied to said first plastic film layer (12) by lithographic printing.

11. A laminated structure (10) as claimed in any one of the preceding claims, wherein the silicon oxide layer (16) is adhered to the or each clear plastic layer (12, 14), through the interposition of suitable tie coats (18, 20) such as clear polyurethane or the like.

12. A packaging arrangement for the sealed containment of a plurality of hydrophilic contact lenses each in a sterile aqueous solution; comprising:
a) a plurality of molded plastic base members (28) contiguously positioned in an array (32), each said base member (28) having a cavity (40) for containing one contact lens immersed in said aqueous solution, and each said base member (28) including a substantially planar flange (35) extending outwardly about the periphery of said cavity (40); and
b) a unitary generally transparent flexible cover sheet (10) according to any one of Claims 1 to 11 extending superimposed over said plurality of base members (28) and being detachably sealed to the surface of said flanges (35) at least about the rim of each said cavity (40), said cover sheet (10) comprising a transparent outer layer (12) imprinted to define a specified pattern of indicia and legends extending spread over the surface thereof, said laminate (10) being separable into segments each forming a cover for respectively one therewith associated base member (28), whereby separation of at least one said segment from the remaining laminate provides visual evidence of separation from said array (32) upon inspection of said printing pattern.

13. A packaging arrangement as claimed in Claim 12, wherein weakening lines (34) are formed in said laminated structure (10) to facilitate separation into said segments, said pattern of indicia and legends being printed on the structure extending over said weakening lines (34) such that predetermined regions of said printing are divided at said weakening lines (34) to form indication of discontinuities in said printing pattern upon separation of said segments.

14. A packaging arrangement as claimed in Claim 13, wherein said weakening lines (34) comprise perforations cut to extend at least partially through said laminate material (10).

15. A packaging arrangement as claimed in any one of Claims 12 to 14, wherein said outer plastic film layer (12) is imprinted on both surfaces thereof, a surface of said film layer opposite said first imprinted surface being imprinted with further indicia at specific regions over each respective container.

16. A packaging arrangement as claimed in Claim 15, wherein said further indicia is imparted to the outer surface of said outer plastic film layer (12) by thermal transfer printing.

17. A packaging arrangement as claimed in any one of Claims 12 to 16, wherein the further plastic film layer (14) is adhesively fastened to the surface of said barrier layer (16) opposite the surface thereof having the outer plastic film layer (12) adhesively fastened thereto.

18. A packaging arrangement as claimed in any one of Claims 12 to 17, wherein said silicon oxide barrier layer (16) inhibits the transmission therethrough of oxygen, water and bacteria while enabling the passage therethrough of light.

19. A packaging arrangement as claimed in Claim 18, wherein said light comprises ultraviolet energy for sterilizing the contents of said packaging arrangement.

## Patentansprüche

1. Klare laminierte Kunststoffmaterialstruktur (10) mit Beschriftungen und versiegelnden Abdeckungen für eine Vielzahl von Kunststoffbehältern (28), die unmittelbar aufeinanderfolgend in einer Anordnung positioniert sind, um ein Produkt innerhalb jedem der Behälter (28) zu versiegeln, wobei die Materialstruktur (10) eine erste klare Kunststoff-Filmschicht (12), die aus Polyester oder einem Polyolefin aufgebaut ist, welches mit einem bestimmten Muster aus Stempelungen und Aufschriften bedruckt ist; eine zweite klare Kunststoff-Filmschicht (14) aus Polypropylen oder Polyethylen, die adhäsiv an einem Basiselement jedes der Behälter zu befestigen ist; und eine Sperrschicht (16) aus Siliciumoxid auf wenigstens einer der ersten und zweiten klaren Kunststoffschichten (12, 14) und adhäsiv daran befestigt, um die laminierte Materialstruktur (10) zu bilden, aufweist.

2. Laminierte Struktur (10) nach Anspruch 1, bei der die Sperrschicht (16) zwischen die erste und die zweite klare Kunststoffschicht (12, 14) gelegt ist.

3. Laminierte Struktur (10) nach Anspruch 1 oder Anspruch 2, bei der die erste Schicht (12) eine äußere Materialschicht aus Polyesterfilm ist, und sie hat eine Fläche (24), die adhäsiv an einer zugewandten Fläche der Sperrschicht (16) zu befestigen ist, wobei das Muster aus bestimmten Stempelungen und Aufschriften auf die Fläche (24) des Filmmaterials (12) aus Polyester gedruckt ist, die der Sperrschicht (16) zugewandt ist.

4. Laminierte Struktur (10) nach Anspruch 1, Anspruch 2 oder Anspruch 3, bei der die erste Filmschicht (12) auf ihren beiden Flächen bedruckt ist, wobei eine Außenfläche der ersten Filmschicht (12) gegenüber der ersten bedruckten Fläche mit weiteren Stempelungen auf bestimmten Bereichen bedruckt ist.

5. Laminierte Struktur (10) nach Anspruch 4, bei der die weiteren Stempelungen auf die Außenfläche der ersten Kunststoff-Filmschicht (12) durch thermischen Transferdruck aufgegeben werden.

6. Laminierte Struktur (10) nach einem der voranstehenden Ansprüche, bei der die klare laminierte Kunststoffmaterialstruktur (10) in ihrer Gesamtheit die Beschriftungen und versiegelnde Abdeckung für die Anordnung bildet, die aus einer Vielzahl der unmittelbar aufeinanderfolgend angeordneten Behälter (28) besteht; wobei Schwächungslinien (34) in der laminierten Materialstruktur (10) gebildet sind, um die Auftrennung in Segmente zu erleichtern, die jeweils aus einem einzelnen Behälter (28) bestehen, wobei das Muster aus Stempelungen und Aufschriften, das auf die Mateiralstruktur (10) gedruckt ist, sich über die Schwächungslinien (34) erstreckt, so daß vorbestimmte Bereiche der Bedruckung an der Schwächungslinie (34) geteilt werden, um eine Anzeige von Diskontinuitäten in dem Druckmuster nach dem Trennen der Segmente zu bilden.

7. Laminierte Struktur (10) nach Anspruch 6, bei der jede der Schwächungslinien (34) Perforationen aufweiset, die sich wenigstens teilweise durch die laminierte Materialstruktur (10) erstrecken, um das Trennen der Struktur in Segmente entlang der Schwächungslinien (34) zu ermöglichen, wobei jedes der Segmente eine Abdeckung für jeweils einen der Behälter (28) bildet.

8. Laminierte Struktur (10) nach Anspruch 6 oder Anspruch 7, bei der die Anordnung der unmittelbar aufeinanderfolgend angeordneten Behälter (28) eine lineare Anordnung aufweist, die aus einer bestimmten Anzahl der Behälter (28) besteht, wobei die laminierte Materialstruktur (10) eine im allgemeine rechtwinklige Konfiguration zum Abdecken der Anordnung hat.

9. Laminierte Struktur (10) nach Anspruch 6, Anspruch 7 oder Anspruch 8, bei der das Druckmuster aus Stempelungen und Aufschriften so ausgestaltet ist, daß es ein schützendes Druckmuster bildet, welches eine Fehldarstellung des Produktes in den Behältern (28) zu unterbindet.

10. Laminierte Struktur (10) nach einem der voranstehenden Ansprüche, bei der das Druckmuster auf die erste Kunststoff-Filmschicht (12) durch lithographisches Drucken aufgebracht wird.

11. Laminierte Struktur (10) nach einem der voranstehenden Ansprüche, bei der die Siliciumoxidschicht (16) an der oder jeder klaren Kunststoffschicht (12, 14) durch das Zwischenschalten geeigneter Haftbeschichtungen (18, 20), so wie klarem Polyurethan oder dergleichen, anhaftet.

12. Verpackungsanordnung für das versiegelte Halten einer Vielzahl hydrophiler Kontaktlinsen jeweils in einer sterilen wäßrigen Lösung; mit:
a) einer Vielzahl geformter Kunststoff-Basiselemente (28), die unmittelbar aufeinanderfolgend in einer Anordnung (32) positioniert sind, wobei jedes Basiselement (28) einen Hohlraum (40) zum Halten einer Kontaktlinse, eingetaucht in die wäßrige Lösung, hat, und jedes der Basiselemente (28) einen im wesentlichen planaren Flansch (35) umfaßt, der sich nach außen um den Umfang des Hohlraums (40) erstreckt; und
b) einer einheitlichen im allgemeinen transparenten flexiblen Deckfolie (10) nach einem der Ansprüche 1 bis 11, die sich über die Vielzahl der Basiselemente (28) gelegt erstreckt und lösbar an der Oberfläche der Flansche (35) wenigstens um den Rand jedes Hohlraums (40) versiegelt ist, wobei die Deckfolie (10) eine transparente äußere Schicht (12) aufweist, die bedruckt ist, um ein bestimmtes Muster aus Stempelungen und Aufschriften zu definieren, das sich über die Oberfläche verstreut erstreckt, wobei das Laminat (10) in Segmente auftrennbar ist, die jedes eine Abdeckung für jeweils ein zugewiesenes Basiselement (18) bildet, so daß die Trennung wenigstens eines der Segmente von dem verbleibenden Laminat einen visuellen Nachweis der Trennung von der Anordnung (32) nach Überprüfung des Druckmusters bietet.

13. Verpackungsanordnung nach Anspruch 12, bei der Schwächungslinien (34) in der laminierten Struktur (10) gebildet sind, um die Auftremung in die Segmente zu erleichtem, wobei das Muster aus Stempelungen und Aufschriften, das auf die Struktur gedruckt ist, sich über die Schwächungslinien (34) derart erstreckt, so daß vorbestimmte Bereiche der Bedruckung an den Schwächungslinien (34) geteilt werden, um eine Anzeige für Diskontinuitäten in dem Druckmuster nach dem Trennen der Segmente zu bilden.

14. Verpackungsanordnung nach Anspruch 13, bei der Schwächungslinien (34) Perforationen aufweisen, die so geschnitten sind, daß sie sich wenigstens teilweise durch das Laminatmaterial (10) erstrecken.

15. Verpackungsanordnung nach einem der Ansprüche 12 bis 14, bei der die äußere Kunststoff-Filmschicht (12) auf ihren beiden Flächen bedruckt ist, wobei eine Fläche der Filmschicht gegenüber der ersten bedruckten Fläche mit weiteren Stempelungen an bestimmten Bereichen über jedem jeweiligen Behälter bedruckt ist.

16. Verpackungsanordnung nach Anspruch 15, bei der die weiteren Stempelungen auf die Außenfläche der äußeren Kunststoff-Filmschicht (12) durch thermischen Transferdruck aufgegeben sind.

17. Verpackungsanordnung nach einem der Ansprüche 12 bis 16, bei der die weitere Kunststoff Fihnschicht (14) adhäsiv an der Oberfläche der Sperrschicht (16) gegenüber dessen Oberfläche, an der die äußere Kunststoff-Filmschicht (12) adhäsiv befestigt ist, befestigt ist.

18. Verpackungsanordnung nach einem der Ansprüche 12 bis 17, bei der die Sperrschicht (16) aus Siliciumoxid den Durchlaß von Sauerstoff, Wasser und Bakterien verhindert, während der Durchlaß von Licht ermöglicht ist.

19. Verpackungsanordnung nach Anspruch 18, bei der das Licht Ultraviolettenergie zum Sterilisieren der Inhalte der Verpackungsanordnung aufweist.

## Revendications

1. Structure en matière plastique feuilletée transparente (10) comprenant des étiquettes et des couvercles d'étanchéité pour une pluralité de récipients plastiques (28) positionnés de manière contiguë dans un ensemble pour tenir de façon étanche un produit à l'intérieur de chacun desdits récipients (28), ladite structure matérielle (10) ayant une première couche plastique en pellicule transparente (12) constituée en polyester ou d'une polyoléfine qui est imprimée avec un motif spécifié d'indices et de légendes ; une seconde couche plastique en pellicule transparente (14) en polypropylène ou en polyéthylène qui peut être fixée de manière adhésive à un organe de base de chacun des récipients ; et une couche barrière (16) en oxyde de silicium sur au moins l'une desdites première et seconde couches plastiques transparentes (12, 14) et fixée de manière adhésive à celle(s)-là pour former ladite structure matérielle feuilletée (10).

2. Structure feuilletée (10) comme revendiqué dans la revendication 1, dans laquelle ladite couche barrière (16) est interposée entre lesdites première et seconde couches plastiques transparentes (12, 14).

3. Structure feuilletée (10) comme revendiqué dans la revendication 1 ou dans la revendication 2, dans laquelle ladite première couche (12) est une couche de matériau en film de polyester externe et elle a une surface (24) qui peut être fixée de manière adhésive à une surface en regard de ladite couche barrière (16), ledit motif d'indices et de légendes spécifiés étant imprimé sur la surface (24) dudit matériau en film de polyester (12) faisant face à ladite couche barrière (16).

4. Structure feuilletée (10) comme revendiqué dans la revendication 1, la revendication 2 ou la revendication 3, dans laquelle ladite première couche en film (12) est imprimée sur les deux surfaces de celle-là, une surface externe de ladite première couche en film (12) opposée à ladite première surface imprimée étant imprimée avec des indices supplémentaires à des régions spécifiques de celle-là.

5. Structure feuilletée (10) comme revendiqué dans la revendication 4, dans laquelle lesdits indices supplémentaires sont répartis sur la surface externe de ladite première couche en film plastique (12) par une impression par transfert thermique.

6. Structure feuilletée (10) comme revendiqué dans l'une quelconque des revendications précédentes, dans laquelle ladite structure en matière plastique feuilletée, transparente (10) forme communément ladite étiquette et ledit couvercle d'étanchéité pour ledit ensemble se composant d'une pluralité desdits récipients disposés de manière contiguë (28) ; des lignes d'affaiblissement (34) étant formées dans ladite structure matérielle feuilletée (10) pour faciliter une séparation en segments, chacun se composant d'un seul récipient (28), ledit motif d'indices et de légendes étant imprimé sur la structure matérielle (10) s'étendant sur lesdites lignes d'affaiblissement (34) de telle sorte que des régions prédéterminées de ladite impression sont divisées auxdites lignes d'affaiblissement (34) pour former une indication des discontinuités dans ledit motif d'impression lors de la séparations desdits segments.

7. Structure feuilletée (10) comme revendiqué dans la revendication 6, dans laquelle lesdites lignes d'affaiblissement (34) comprennent chacune des perforations s'étendant au moins partiellement à travers ladite structure matérielle feuilletée (10) pour permettre de séparer ladite structure en des dits segments le long desdites lignes d'affaiblissement (34), lesdits segments formant chacun un couvercle pour respectivement un dit récipient (28).

8. Structure feuilletée (10) comme revendiqué dans la revendication 6 ou la revendication 7, dans laquelle ledit ensemble de récipients disposés de manière contiguë (28) comprend un ensemble linéaire se composant d'un nombre spécifié desdits récipients (28), ladite structure matérielle feuilletée (10) ayant une configuration généralement rectangulaire pour recouvrir ledit ensemble.

9. Structure feuilletée (10) comme revendiqué dans la revendication 6, la revendication 7 ou la revendication 8, dans laquelle ledit motif d'impression des indices et de légendes est configuré pour former un motif d'impression de protection gênant la déformation du produit dans lesdits récipients (28).

10. Structure feuilletée (10) comme revendiqué dans l'une quelconque des revendications précédentes, dans laquelle ledit motif d'impression est appliqué à ladite première couche en film plastique (12) par une impression lithographique.

11. Structure feuilletée (10) comme revendiqué dans l'une quelconque des revendications précédentes, dans laquelle la couche en oxyde de silicium (16) est collée à la ou à chaque couche plastique transparente (12, 14) grâce à l'interposition de couches de raccordement (18, 20) telles que le polyuréthane transparent ou analogue.

12. Arrangement de conditionnement pour le confinement étanche d'une pluralité de lentilles de contact hydrophiles, chacune dans une solution aqueuse stérile ; comprenant :
a) une pluralité d'organes de base en plastique moulés (28) positionnés de manière contiguë dans un ensemble (32), chaque dit organe de base (28) ayant une cavité (40) pour contenir une lentille de contact immergée dans ladite solution aqueuse, et chaque dit organe de base (28) incluant un rebord sensiblement plan (35) s'étendant vers l'extérieur autour de la périphérie de ladite cavité (40) ; et
b) une feuille de couverture flexible, unitaire, généralement transparente (10) selon l'une quelconque des revendications 1 à 11 s'étendant de façon superposée sur ladite pluralité d'organes de base (28) et étant, de manière détachable, scellée à la surface desdits rebords (35) au moins autour du bord de chaque dite cavité (40), chaque feuille de couverture (10) comprenant une couche externe transparente (12) imprimée pour définir un motif spécifié d'indices et de légendes s'étendant de façon étendue sur la surface de celle-là, ledit stratifié (10) étant séparable en segments, chacun formant un couvercle pour respectivement un organe de base associé à celui-là (28), de sorte que la séparation d'au moins un dit segment du stratifié restant offre une évidence visuelle de séparation dudit ensemble (32) lors de l'inspection dudit motif d'impression.

13. Arrangement de conditionnement comme revendiqué dans la revendication 12, dans lequel les lignes d'affaiblissement (34) sont formées dans ladite structure feuilletée (10) pour faciliter la séparation en des dits segments, ledit motif d'indices et de légendes étant imprimé sur la structure s'étendant sur lesdites lignes d'affaiblissement (34) de telle sorte que des régions prédéterminées de ladite impression sont divisées auxdites lignes d'affaiblissement (34) pour former une indication des discontinuités dans ledit motif d'impression lors de la séparations desdits segments.

14. Arrangement de conditionnement comme revendiqué dans la revendication 13, dans lequel lesdites lignes d'affaiblissement (34) comprennent des perforations découpées pour s'étendre au moins partiellement à travers ledit matériau feuilleté (10).

15. Arrangement de conditionnement comme revendiqué dans l'une quelconque des revendications 12 à 14, dans lequel ladite couche en film plastique externe (12) est imprimé sur les deux surfaces de celle-là, une surface de ladite couche en film opposée à ladite première surface imprimée étant imprimée avec des indices supplémentaires à des régions spécifiques sur chaque récipient respectif.

16. Arrangement de conditionnement comme revendiqué dans la revendication 15, dans lequel lesdits indices supplémentaires sont répartis sur la surface externe de ladite couche en film plastique externe (12) par une impression par transfert thermique.

17. Arrangement de conditionnement comme revendiqué dans l'une quelconque des revendications 12 à 16, dans lequel la couche en film plastique supplémentaire (14) est fixée de manière adhésive à la surface de ladite couche barrière (16) opposée à sa surface ayant la couche en film plastique externe (12) fixée de manière adhésive à celle-là.

18. Arrangement de conditionnement comme revendiqué dans l'une quelconque des revendications 12 à 17, dans lequel ladite couche barrière en oxyde de silicium (16) gêne la transmission à travers elle de l'oxygène, de l'eau et des bactéries tout en permettant le passage à travers elle de la lumière.

19. Arrangement de conditionnement comme revendiqué dans la revendication 18, dans lequel ladite lumière comprend l'énergie ultraviolette pour stériliser le contenu dudit arrangement de conditionnement.
